# EUROPEAN PATENT SPECIFICATION

(11) **EP 3 711 727 B1**
(45) Date of publication and mention of the grant of the patent: **04.08.2021**
(21) Application number: 20164718.7
(22) Date of filing: 20.03.2020
(51) Int. Cl.: A61F 13/15, A61F 13/49, A61F 13/537, A61F 13/539, A61F 13/56

(54) **REUSABLE INCONTINENCE MATERIAL**
WIEDERVERWENDBARES INKONTINENZMATERIAL
MATÉRIAU CONTRE L'INCONTINENCE RÉUTILISABLE

(30) Priority: 21.03.2019 BE 201905177
(43) Date of publication of application: 23.09.2020
(73) Proprietor: Washcot bv, 8800 Roeselare (BE)
(72) Inventor: VANDENBOGAERDE, David, 8540 Deerlijk (BE); LAMBERT, Isabel, 8540 Deerlijk (BE)
(74) Representative: DenK iP bv

(56) References cited:
- WO-A1-95/12374
- WO-A1-2010/141643

## Description

### Field of the invention

The invention generally concerns incontinence material, such as for example nappies. More specifically, the invention relates for instance to reusable nappies.

### Background of the invention

Incontinence material is available at present in various forms. Both single-use nappies and reusable nappies are offered, also single-use underpants and reusable underpants. Single-use underpants are often made of the same material as single-use nappies, but have a different shape. Single-use nappies and underpants have the disadvantage that they take a long time to decompose.

Reusable nappies and underpants offer an environmentally-friendly alternative to single-use nappies and underpants. Like single-use nappies and underpants, with reusable nappies and underpants it is important that they fit comfortably. It is also important that the chance of leakage is as small as possible.

In addition, it is important that the reusable nappies and underpants have good absorption capacity. For this, absorbent materials may be attached to the nappy or underpants. Reusable underpants which serve as incontinence material are often cotton underpants in which a pad of absorbent material is attached at the bottom. It is essential that these absorbent materials, and the other materials from which the nappy or underpants are made, are washable.

Because of the various requirements applicable to a reusable nappy or underpants, including comfortable fit, good absorption capacity and washability, there is room for good reusable incontinence material and ways of making this.

WO 95/12374 A1 discloses a washable nappy comprising a layered structure with a top sheet, an absorbent core and an impermeable back sheet. The top sheet, absorbent core and a support layer are joined together via central seams by stitching. The folded-back edges of the top and back sheets are stitched together along peripheral seams in regions intended to be in contact with the legs of the user. The support layer is not capable of acting as a distribution layer since it is located on the side of the absorbent core facing the back sheet,

### Summary of the invention

It is an object of embodiments of the present invention to provide good reusable incontinence material, in particular for example nappies, and also a method for making such incontinence material.

The above objective is achieved by material and/or a method according to the present invention.

In a first aspect, the present invention provides reusable incontinence material, such as for example nappies and (trainer) underpants and incontinence pads for use in underpants, comprising or consisting of a layered structure, as defined in claim 1.

The reusable incontinence material has an inner side interned to be oriented towards, or even to be in contact with, the body of a wearer, and an outer side intended to be oriented away from the body of a wearer. The layered structure comprises at least a contact layer intended to be oriented towards or come into contact with the skin, a dispersion layer, at least one absorption layer, and an impermeable layer intended to form an outer side of the incontinence material. In particular embodiments, more than one absorption layer may be provided, depending on the amount of liquid anticipated to be collected, and taking into account the type of absorption layer used. The different layers are attached to each other by means of one or more middle stitchings and side stitchings, wherein the contact layer, the dispersion layer and the one or more absorption layers are connected together by means of the middle stitchings, wherein the middle stitchings do not pass through the impermeable layer at the outer side of the reusable incontinence material. The incontinence material has an inside (where different layers are in contact with each other) and an outside (intended to be in contact with the user's skin or the environment). In the incontinence material, the side stitchings connect together the contact layer and the impermeable layer without passing through the layers on the outer side of the incontinence material, resulting in two outside edges which can be arranged along the legs of a wearer.

It is an advantage of embodiments of the present invention that the dispersion layer of the layered structure ensures a good spread of fluid, in particular urine, over the absorption layer(s), whereby the fluid can be absorbed evenly in the absorption layer(s). This avoids local saturation of the absorption layer(s).

It is an advantage of embodiments of the present invention that a distinction is made between middle stitchings and side stitchings, and that both are used to form the reusable incontinence material, for example a reusable nappy. The dispersion layer and the absorption layer(s) are attached to the contact layer by means of the middle stitchings. These middle stitchings do not pass through the impermeable layer. Because the stitches do not pass through completely, accordingly less leakage occurs than in incontinence material, e.g. nappies, with complete through-stitching. These middle stitchings hold the dispersion layer and the absorption layer(s) in place. This may avoid mechanical friction between the layers.

The middle stitchings offer a rapid path for conducting moisture to the absorption layer(s). Because they do not pass through the impermeable layer, leakage via the middle stitchings is avoided.

The side stitchings, however, are arranged through the contact layer and the impermeable layer, without passing through the impermeable layer at the outer side of the incontinence material, e.g. the nappy. Potential fluid transport along the side stitchings goes to the inside of the incontinence material, but does not reach the outer side, as these stitchings do not go through the impermeable layer towards the outer side.

In particular embodiments, the side stitchings are arranged through inwardly folded ends of the contact layer and the impermeable layer, without passing through these layers (impermeable layer and contact layer) to the outside of the incontinence material, e.g. the nappy. Since in this case the seam sits on the inside of the incontinence material, e.g. the nappy, fluid transport along the side stitchings through the impermeable layer does not lead to leakage.

Because the formed seams are oriented towards the inside of the incontinence material, e.g. the nappy, they also do not form a nuisance to the wearer.

In reusable incontinence material according to embodiments of the present invention, the absorption layer may be a fleece layer. It is an advantage of a fleece layer, which is neither woven nor knitted, that the incontinence material, e.g. the nappy, can be made thinner than and yet have the same absorption capacity as a woven or knitted layer.

It is an advantage of embodiments of the present invention that this fleece layer is attached to the contact layer by means of middle stitchings. In this way, the one or more absorption layers are held in place. In addition, the fleece absorption layer is encapsulated between the contact layer and the impermeable layer. In this way, a stable fleece layer is obtained.

In reusable incontinence material according to embodiments of the present invention, the side edges of the dispersion layer may be folded over between the dispersion layer and the absorption layer(s), and firmly stitched by means of one or more middle stitchings. It is an advantage of embodiments of the present invention that the folded edge of the dispersion layer pushes moisture back towards the inside, whereby it can be absorbed by the absorption layer(s).

In reusable incontinence material according to embodiments of the present invention, the dispersion layer and the absorption layer(s) may be narrower than the distance between the side stitchings, measured over the contact layer or the impermeable layer. This prevents the absorbent part of the incontinence material, which at least consists of the dispersion layer and the absorption layer(s), from extending to the edge of the incontinence material, e.g. the nappy. By doing this, a less rigid incontinence material is obtained, e.g. a less rigid nappy, than if the absorbent part extends to the edge.

In reusable incontinence material according to embodiments of the present invention, the impermeable layer may comprise or consist of textile material. The textile material may comprise a woven or knitted polyester fabric. The textile material may be laminated. The textile material may be coated with a water-impermeable membrane.

In reusable incontinence material according to embodiments of the present invention, the one or more absorption layers may comprise polyacrylic acid and non-woven polyester.

In reusable incontinence material according to embodiments of the present invention, the contact layer may be a fleece layer. It is an advantage of embodiments of the present invention that a drier feel is created by a rapidly absorbing contact layer.

Reusable incontinence material according to embodiments of the present invention may for example be provided in the form of a nappy, underpants or a pad for use in underpants.

In a second aspect, the present invention provides a method for producing reusable incontinence material which comprises at least a contact layer intended to come into contact with the skin, a dispersion layer, at least one absorption layer, and an impermeable layer intended to form the outside of the incontinence material. The method comprises:
- stitching the contact layer to the dispersion layer and the at least one absorption layer by means of one or more middle stitchings,
- stitching the contact layer to the impermeable layer along two outside edges of these layers, wherein during stitching, the dispersion layer and the absorption layer(s) are on the outside of a volume enclosed by the contact layer and impermeable layer, and
- turning the layers inside out so that the dispersion layer and the absorption layer(s) are on the inside of the volume enclosed by the contact layer on the impermeable layer.

By this method, a structure is obtained in which the absorption layer is fixed by being firmly stitched to the contact layer. In addition, these stitchings do not pass through the impermeable layer, thus avoiding leaks. In systems in which the stitchings pass through the absorption layer and also through the impermeable layer, moisture can still leak away via the stitchings. Because the stitchings sit on the inside at the edges, a more comfortable incontinence material is obtained, e.g. a more comfortable nappy.

In a method according to embodiments of the present invention, the dispersion layer and the absorption layer(s) may be narrower than the contact layer and the impermeable layer, and are not stitched in when the contact layer is stitched to the impermeable layer.

Specific and preferred aspects of the invention are contained in the attached independent and dependent claims. Features of the dependent claims may be combined with features of the independent claims, and with features of other dependent claims, as indicated and not merely as expressly presented in the claims.

These and other aspects of the invention will become clear and be explained with reference to the embodiment(s) described below.

### Brief description of the drawings

FIG. 1 shows a diagrammatic, perspective drawing of a nappy in accordance with a first embodiment of the present invention.
FIG. 2 shows a diagrammatic drawing of an inside of a nappy in accordance with the first embodiment of the present invention.
FIG. 3 shows a diagrammatic drawing of the cross-section of a nappy in accordance with embodiments of the present invention.
FIG. 4 shows a diagrammatic drawing of the outside of a nappy in accordance with the first embodiment of the present invention.
FIG. 5 shows an enlarged view of the back of a nappy as shown in FIG. 4, in accordance with embodiments of the present invention.
FIG. 6 shows a diagrammatic, perspective drawing of a nappy in accordance with a second embodiment of the present invention.
FIG. 7 shows a diagrammatic drawing of an inside of a nappy in accordance with the second embodiment of the present invention.
FIG. 8 shows a diagrammatic drawing of an outside of a nappy in accordance with the second embodiment of the present invention.
FIG. 9 shows a diagrammatic, perspective drawing of a nappy in accordance with a third embodiment of the present invention.
FIG. 10 shows a diagrammatic drawing of an inside of a nappy in accordance with the third embodiment of the present invention.
FIG. 11 shows a diagrammatic drawing of an outside of a nappy in accordance with the third embodiment of the present invention.FIG. 12 shows a diagrammatic drawing of an inside of a nappy in accordance with the second embodiment of the present invention.

The figures are purely diagrammatic and not limitative. In the figures, the dimensions of some components may be exaggerated and not shown to scale for illustrative purposes.

Reference numbers in the claims should not be interpreted as restricting the scope of protection. In the various figures, the same reference numbers refer to the same or equivalent elements.

### Detailed description of illustrative embodiments

The present invention will be described in relation to particular embodiments and with reference to specific drawings, but the invention is not restricted thereto and is limited solely by the claims. The drawings described are purely diagrammatic and not limitative. In the drawings, for illustrative purposes, the dimensions of some elements are exaggerated and not drawn to scale. The dimensions and relative dimensions sometimes do not correspond to the actual practical embodiment of the invention.

It should be noted that the term "comprise" as used in the claims should not be interpreted as limited to the means described thereafter; these terms do not exclude other elements or steps. They should be interpreted as specifying the presence of the indicated features, values, steps or components to which reference is made, but do not exclude the presence or addition of one or more other features, values, steps or components or groups thereof. Therefore the scope of the expression "a device comprising means A and B" should not be limited to devices which consist solely of components A and B. It means that with respect to the present invention, A and B are the only relevant components of the device.

Reference throughout this specification to "one embodiment" or "an embodiment" means that a specific feature, structure or characteristic described in connection with the embodiment is included in at least one embodiment of the present invention. Therefore the expressions "in one embodiment" or "in an embodiment" at various points throughout the specification need not necessarily each refer to the same embodiment, but may well do so. Furthermore, the specific features, structures or characteristics may be combined in any suitable manner in one or more embodiments, as will be clear to an average person skilled in the art on the basis of this publication.

Similarly, it should be appreciated that the various features of the invention given in the description of exemplary embodiments of the invention may sometimes be grouped into a single embodiment, figure or description thereof, with the aim of streamlining the disclosure and assisting with comprehension of one or more of the various inventive aspects. This method of disclosure should not be interpreted as reflecting an intention that the invention requires more features than explicitly stated in each claim. Rather, as the following claims show, inventive aspects exist in less than all features of a single, previously disclosed embodiment. Therefore, the claims following the detailed description are hereby explicitly included in this detailed description, with each autonomous claim as a separate embodiment of this invention.

Furthermore, while some embodiments described herein comprise some but not other features included in other embodiments, combinations of features of different embodiments are considered to lie within the scope of the invention and form different embodiments, as will be understood by the skilled person. For example, in the claims which follow, any embodiments described may be used in any combination.

In the description provided here, numerous specific details are emphasised. It should however also be understood that embodiments of the invention may be implemented without these specific details. In other cases, well-known methods, structures and techniques are not presented in detail, in order to keep this description concise.

In particular, the detailed description given below refers to nappies as a specific example of incontinence material. The present invention is not however restricted thereto and also includes underpants which are constructed in the same manner but have a different shape. Pads of incontinence material, which can be placed in underpants and have the same composition, also fall under the scope of protection of the present invention.

In the context of the present invention, the terminology "inner side" and "outter side" is used, as well as the terminology "inside" and outside". The inside of the incontinence material is that side where the dispersion and absorption layers are present, while the outside is that side which is facing the environment. Basically, the incontinence material is a substantially flat 3D structure, which has first and second opposite major surfaces at the outside thereof. The first major surface may be the inner side of the incontinence material, intended to be oriented towards, or even to be in contact with, the body of a wearer, while the second major surface may be the outer side of the incontinence material, intended to be oriented away from the body of the wearer.

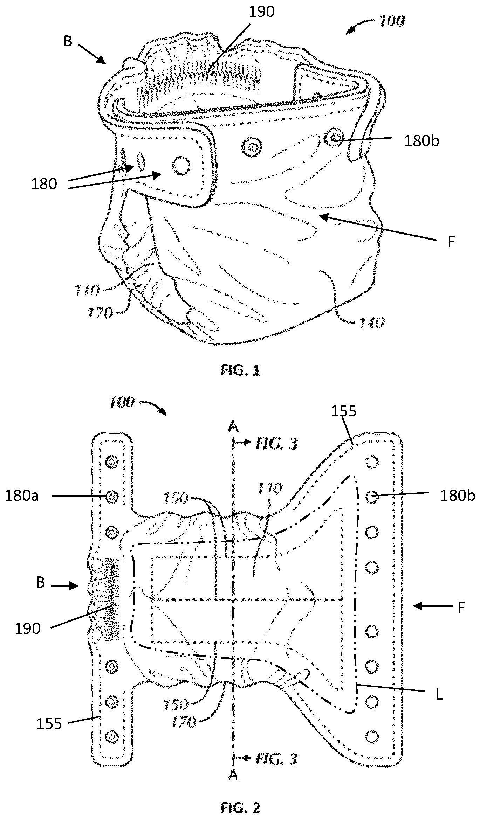

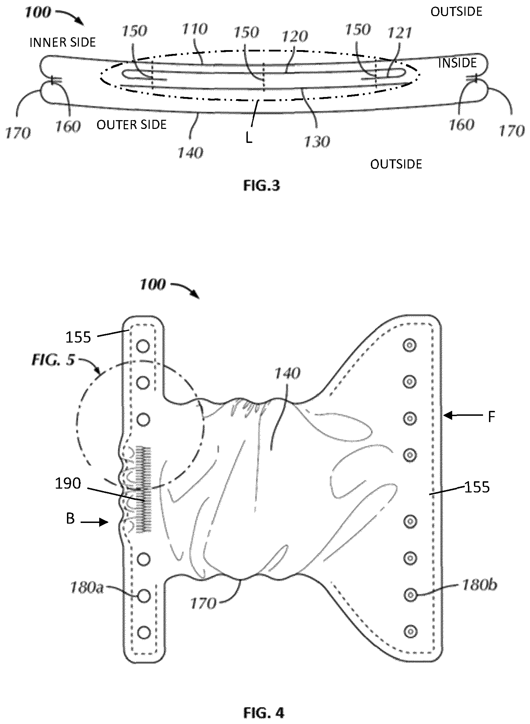

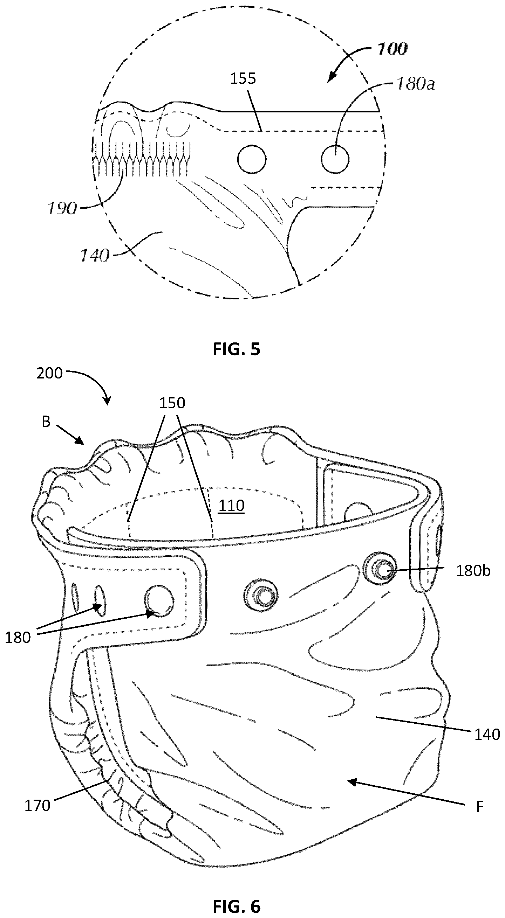

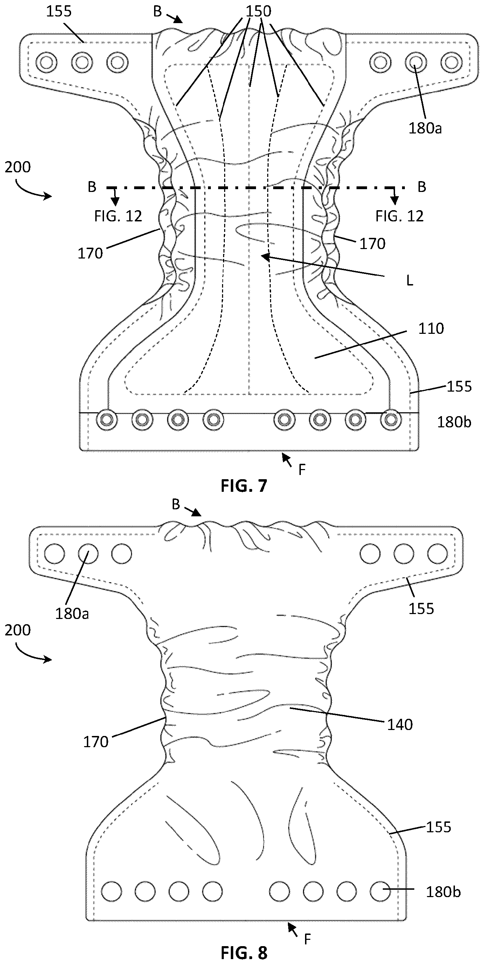

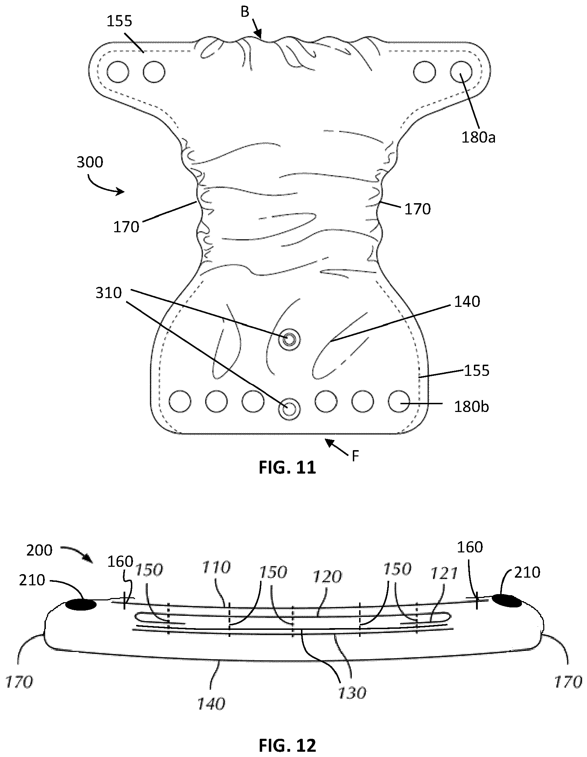

In a first aspect, embodiments of the present invention relate to reusable incontinence material in the form of a reusable nappy 100, 200, 300. This reusable nappy comprises a layered structure which contains a contact layer 110, a dispersion layer 120, one or more absorption layers 130 and an impermeable layer 140. The layers are attached to each other by means of middle stitchings 150 and side stitchings 160. There where the dispersion layer and the one or more absorption layers are present, a liquid collecting area is formed. FIGs. 1, 2, 4 and 5 show perspective drawings of a nappy in accordance with a first embodiment of the present invention, while FIGs. 6 to 8 and 9 to 11 show different views of a nappy according to a second and a third embodiment, respectively. The reference numbers used in these figures correspond to each other in the different figures. FIG. 3 and FIG. 12 show a diagrammatic drawing of the cross-section of different embodiments of nappies. The location of the cross-section AA and BB is shown in FIG. 2, and in FIGs. 7, respectively.

In embodiments of the present invention, the contact layer 110, the dispersion layer 120 and the absorption layer(s) 130 are connected together by means of one or more middle stitchings 150. These middle stitchings do not pass through the impermeable layer 140. FIG. 2, FIG. 3 and FIG. 10 show 3 middle stitchings, while FIG. 7 and FIG. 13 show 5 middle stitchings. In the embodiment illustrated, the stitchings run substantially parallel to the edges of the nappy which can be arranged around the legs of the wearer. This is not, however, strictly necessary. The middle stitchings may for example also run diagonally or follow an arbitrary pattern. The invention is also not restricted to 3 or 5 middle stitchings, not even to an uneven number of middle stitchings 150. In some embodiments for example (not illustrated), only one middle stitching is present, and in some embodiments for example, 2 or 3 or more than 3 middle stitchings may be present. The number of middle stitchings applied may be determined by the size of the diaper and/or the required stability of the layers.

In embodiments of the present invention, the contact layer and the impermeable layer have a three-dimensional structure.

In embodiments of the present invention, the contact layer 110 is a structurally rigid layer which is attached by means of side stitchings 160 to the impermeable layer 140. In doing so, the inwardly folded ends of the contact layer 110 and the impermeable layer 140 are connected to each other without the side stitchings passing through the layers of the outside of the nappy. In other words, the unfolded edges of the contact layer and the impermeable layer are stitched through, but the contact layer and the impermeable layer are not stitched through to the outside of the nappy by the side stitchings 160. The side stitchings form the two outside edges 170 which can be arranged around the legs of a wearer.

In this way, a nappy is obtained in which the dispersion layer 120 and the one or more absorption layers 130 are held in place by means of the middle stitchings 150, and wherein the outside edges 170 which must be arranged around the legs of the wearer are produced by means of side stitchings 160 which do not pass through the outside contact layer 110 and impermeable layer 140, and the seam of which is directed towards the inside of the nappy (in the volume between the contact layer and the impermeable layer).

In embodiments of the present invention, the absorption layer 130 is a fleece layer (non-woven), in other words a textile layer which is neither woven nor knitted, for example a fibre fleece or a filament fleece. Such a material consists of arbitrarily oriented fibres. In embodiments of the present invention, the absorption layer is a washable layer. The absorption layer 130 is encapsulated between the contact layer 110 and the impermeable layer 150. In other words, it is held in stable fashion between several layers. Even on repeated washing, the fleece layer will thus not fall apart.

In embodiments of the present invention, a plurality of absorption layers 130 may be stacked onto one another, as for instance illustrated in FIG. 12, possibly with a further dispersion layer in between them (not illustrated). The fact that the plurality of absorption layers 130 is illustrated in the context of the second embodiment only is not limiting for the present invention; a plurality of absorption layers may be, but do not need to be, present in each and every embodiment of the present invention.

In embodiments of the present invention, as illustrated in FIG. 3 and FIG. 12, the side edges 121 of the dispersion layer 120 are folded over before the dispersion layer 120 is stitched to the absorption layer 130. This, however, is not limiting for the present invention, and the dispersion layer 120 could be simply provided in a flat state in the incontinence material. The folded edges are stitched firmly between the dispersion layer 120 and the absorption layer 130. In this way, a double dispersion layer is created at the edge. Fluid on the edge of the dispersion layer is thus conducted away from the edge, in the direction of the centre of the absorption layer, by the folded edge of the dispersion layer. The folded edge may for example have a width of a few millimetres up to half a centimetre or even a few centimetres.

In embodiments of the present invention, the impermeable layer 150 comprises or consists of textile material. The textile material may for example comprise a woven or knitted polyester fabric. It may for example consist of coated textile materials, wherein typically a knitted (or woven) polyester (or polyamide or cotton or viscose) fabric with a light grammage (e.g. between 50 and 300 g/m2) is coated with a polymer coating.

In embodiments of the present invention, the textile material may be laminated. In embodiments of the present invention, the textile material is coated with a water-impermeable membrane. Polymer coatings such as polyurethane, polyvinyl chloride, polyacrylate and polyolefin may be applied.

In embodiments of the present invention, the impermeable layer 150 may also consist of a laminated textile, wherein a water-impermeable membrane (typically polyurethane, polytetrafluoroethylene or polyolefin) is laminated on or between one or more layers of textile (knitted or woven fabric).

In an exemplary embodiment of the present invention, the impermeable layer consists for example of a knitted fabric coated with polyurethane.

In another exemplary embodiment of the present invention, the impermeable layer consists for example of a polyurethane membrane laminated between two knitted fabrics of for example 100 g/m2.

In embodiments of the present invention, the absorption layer comprises for example polyacrylic acid and non-woven polyester. The absorption layer may for example consist to a majority of polypropylene (e.g. 70%) and to a smaller proportion of polyacrylic acid (e.g. 30%).

In an exemplary embodiment of the present invention, the absorption layer consists of highly absorbent, non-woven materials/knitted or woven fabrics with a specific weight between 50 - 300 g/m2, which have a high absorption capacity, typically between 500 and 2500% according to ISO 9073-6. This may for example consist of viscose, cotton, polyester, polyolefin, polyacrylic acid (PAA) or mixtures thereof.

One example is Pelyloft PZ 90/30 by Pely-Tex. This consists of 70% polyester/30% viscose at 90 g/m2 with an absorption of 1000%.

In embodiments of the present invention, the dispersion layer 120 consists for example of water-dispersing, non-woven materials or knitted or woven fabrics with, for example, a specific weight between 50 and 300 g/m2. This may for example consist of viscose, cotton, polyester or mixtures thereof.

In embodiments of the present invention, the contact layer consists for example of non-woven materials or knitted or woven fabrics with a comfortable feel and with, for example, a specific weight between 50 and 300 g/m2. They may for example comprise viscose, cotton, polyester or mixtures thereof. The contact layer may for example be fleece, but also another knitted fabric of for example polyester may be used. Such a layer may for example have a specific weight between 100 and 300 g/m2, for example about 150 g/m2.

FIG. 1 shows a diagrammatic, perspective drawing of a nappy in accordance with a first embodiment of the present invention. The figure shows a top view, front view and side view of the nappy 100. The figure shows the contact layer 110, the impermeable layer 140 and the outside edges 170. The nappy is closed by means of fastening means, such as for instance buttons of press-stud closures, but also any other suitable type of fastening means could be used, such as for instance buttons, or hook and loop fasteners. In the embodiment illustrated, the nappy is closed by means of a number of press-stud closures 180, which provides an easy and convenient way of securing the nappy. A press-stud closure 180 comprises 2 parts: a first part 180a which is a receiving part, and a second part 180b which is a projecting part. In the embodiment illustrated, the first part 180a is made of a cap and socket, and the second part 180b is made of a stud and post, but that is not limiting for the present invention; press-stud closures where the first and second part each only consist of a single item also exist. The first part 180a has a cavity adapted for receiving a projection of the second part 180b. In the embodiment illustrated in FIG. 1, more second parts 180b are present than there are first parts 180a, e.g. eight second parts and only six first parts. This allows a better fit of the nappy, depending on the user's waist circumference; however, this is not limiting for the present invention.

FIG. 2 shows the inside of the same nappy 100. The figure shows the middle stitchings 150, the contact layer 110 and the outside edges 170. The location of the cross-sectional plane AA is also indicated on this figure. First parts 180a and second parts 180b of the press-stud closures 180 are provided at front side F and back side B upper parts of the nappy. An elastic band or stretchable strip 190 is provided at the back side of the nappy 100, for a tight fit in the back of the user.

FIG. 3 shows a diagrammatic drawing of a cross-section along the cross-sectional plane AA indicated in FIG. 2. The contact layer 110, the dispersion layer 120, the absorption layer 130, the impermeable layer 140, the middle stitchings 150, the side stitchings 160, the side edges 121 and the outside edges 170 are shown on this figure.

FIG. 4 shows a diagrammatic drawing of the outside of the same nappy 100. The figure shows the impermeable layer 140. The nappy in the figures can be secured by means of press-stud closures (180a, 180b). The invention is not, however, restricted thereto. Other fixing methods, such as for example hook and loop tape, are also quite possible.

FIG. 5 also shows a diagrammatic drawing of the outside of the same nappy 100. The figure shows the back B of the nappy 100. The impermeable layer 140 can be seen. Also a stretchable strip 190 can be seen. This is however not strictly necessary.

It can be seen in the different drawings illustrating the first embodiment of the nappy 100, that, in this embodiment, outside the liquid collecting area L, e.g. at the belly and the back, the contact layer 110 and the impermeable layer 140 are fastened to one another by means of a fastening stitching 155 which goes through both layers.

This is different from the second and third embodiments of the first aspect of the present invention, in which also the fastening stitchings 155 going through the contact layer 110 and the impermeable layer 140 are reduced. For instance, at the top of the back side B, from comparison of FIG. 8 or FIG. 11 with FIG. 4, it can be readily appreciated that in the second and third embodiments the fastening stitching 155 has been removed, and replaced by a stitching comparable to the stitching 160, i.e. where the stitching does not go through the layers at the outside of the nappy 200, 300.

Furthermore, while in the first embodiment a stretching band 190 is present, this is not the case in the second and third embodiments. However, this is for illustrational purposes only; the stretching band 190 is optional and could be provided or not provided in any of the embodiments.

Another difference which can be appreciated from the drawings illustrating the different embodiments of the nappies 100, 200, 300 is that finishing around the legs (in the neighbourhood of the edge 170) is different for the second and third embodiments compared to the first embodiment. In the second and third embodiments, another stretching band 210 is provided around the legs, while this is not the case in the first embodiment. Again, this presence or non-presence of this further stretching band 210 is not tied to the embodiments, and is for illustrational purposes only; the stretching band 210 around the legs is optional and can be present or not present in any or all of the embodiments. The stretching band 210 around the legs provides a better protection against leakage at that location. The stretching band 210 is preferably attached to the inside of the incontinence material, at the contact layer 110. The stretching band 210 may be stitched to the contact layer 110, or may be attached thereto in any suitable way. If stitched, this stitching then preferably is a stitching which goes through the contact layer and the stretching band 210, but not through the impermeable layer 140, to reduce leakage.

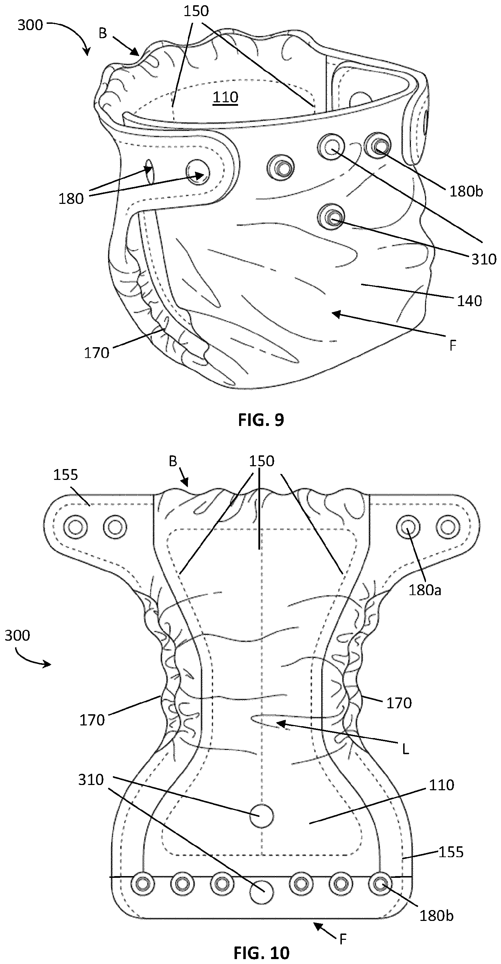

A further difference between the first embodiment of the nappy 100 on the one hand, and the second and third embodiments of the nappies 200, 300 on the other hand, is that no impermeable material 140 is to be found at the side of the nappy 100 of the first embodiment intended to be in contact with the body, while in the second and third embodiments such impermeable material 140 covers a substantial part of the rim of the side of the nappies 200, 300 intended to be in contact with the body. In the particular embodiment illustrated in FIG. 7 and FIG. 10, the impermeable material 140 covers all rims over a predetermined distance, e.g. between 0,5 cm and 3 cm, except the middle of the back.

The third embodiment of the present invention is a nappy for newborns. It differs from the other embodiments in that a supplementary attaching means, e.g. a press-stud closure 310, is provided, which allows to remove the nappy 300 from the belly to give free space to the umbilical cord.

In a second aspect, embodiments of the present invention relate to a method of producing a reusable nappy.

This method comprises stitching of a contact layer to a dispersion layer and at least one absorption layer by means of middle stitchings.

This method also comprises stitching the contact layer to an impermeable layer at the two outside edges of these layers by means of side stitchings. During stitching of these layers, the dispersion layer and the at least one absorption layer are on the outside of the volume enclosed by the contact layer and the impermeable layer.

After stitching of the middle stitchings and side stitchings, in this method a following step is performed. This comprises turning the layers inside out so that the dispersion layer and the at least one absorption layer are on the inside of a volume enclosed by the contact layer and the impermeable layer.

In embodiments of the present invention, the dispersion layer and the at least one absorption layer are not stitched in when the side stitching is stitched. These layers are in this case narrower than the contact layer and the impermeable layer.

The various aspects can easily be combined with each other and the combinations thus also correspond to embodiments according to the present invention.

## Claims

1. Reusable incontinence material (100, 200, 300) having an inner side intended to be oriented towards the body of a wearer, and an outer side intended to be oriented away from the body of a wearer, the reusable incontinence material comprising a layered structure, the layered structure comprising a contact layer (110), a dispersion layer (120), at least one absorption layer (130), an impermeable layer (140), at least one or more middle stitchings (150) and side stitchings (160),
- wherein the contact layer (110), the dispersion layer (120) and the at least one absorption layer (130) are connected together by means of the middle stitchings (150), wherein the middle stitchings (150) do not pass through the impermeable layer (140) at the outer side of the reusable incontinence material,
- and wherein the side stitchings (160) connect together the contact layer (110) and the impermeable layer (140) without passing through the layers on the outer side of the incontinence material, resulting in two outside edges (170) which can be arranged along the legs of a wearer.

2. Reusable incontinence material (100) according to claim 1, wherein the side stitchings (160) connect together inwardly folded ends of the contact layer (110) and the impermeable layer (140).

3. Reusable incontinence material (100) according to any of the previous claims, wherein the absorption layer (130) is a fleece layer.

4. Reusable incontinence material (100) according to one of the preceding claims, wherein side edges (121) of the dispersion layer (120) are folded over between the dispersion layer (120) and the absorption layer (130), and firmly stitched by means of the middle stitching (150).

5. Reusable incontinence material (100) according to any of the preceding claims, wherein the dispersion layer (120) and the absorption layer (130) are narrower than the distance between the side stitchings, measured over the contact layer (110) or the impermeable layer (140).

6. Reusable incontinence material (100) according to any of the preceding claims, wherein the impermeable layer (150) consists of a textile material.

7. Reusable incontinence material (100) according to claim 6, wherein the textile material comprises a woven or knitted polyester fabric.

8. Reusable incontinence material (100) according to one of claims 6 or 7, wherein the textile material is laminated.

9. Reusable incontinence material (100) according to one of claims 6 to 8, wherein the textile material is coated with a water-impermeable membrane.

10. Reusable incontinence material (100) according to any of the preceding claims, wherein the absorption layer contains polyacrylic acid and non-woven polyester.

11. Reusable incontinence material (100) according to any of the preceding claims, wherein the contact layer (110) is a fleece layer.

12. Reusable incontinence material according to any of the preceding claims, in the form of a nappy, underpants or a pad for use in underpants.

13. Method for producing reusable incontinence material which comprises at least a contact layer (110) intended to come into contact with the skin, a dispersion layer (120), at least one absorption layer (130), and an impermeable layer (140) intended to form an outer side of the incontinence material, the method comprising:
- stitching the contact layer (110) to the dispersion layer (120) and the at least one absorption layer (130) by means of one or more middle stitchings (150),
- stitching the contact layer (110) to the impermeable layer (140) along two outside edges of these layers, wherein during stitching the dispersion layer and the absorption layer are on the outside of a volume enclosed by the contact layer and impermeable layer,
- turning the layers inside out so that the dispersion layer and the absorption layer are on the inside of the volume enclosed by the contact layer and the impermeable layer.

14. Method according to claim 13, wherein the dispersion layer and the absorption layer are narrower than the contact layer and the impermeable layer, and are not stitched in when the contact layer is stitched to the impermeable layer.

## Patentansprüche

1. Wiederverwendbares
Inkontinenzmaterial (100, 200, 300) mit einer Innenseite, die dazu bestimmt ist, zum Körper eines Trägers hin ausgerichtet zu sein, und einer Außenseite, die dazu bestimmt ist, vom Körper eines Trägers weg ausgerichtet zu sein, wobei das wiederverwendbare Inkontinenzmaterial eine Schichtstruktur aufweist, wobei die Schichtstruktur eine Kontaktschicht (110), eine Dispersionsschicht (120), mindestens eine Absorptionsschicht (130), eine undurchlässige Schicht (140), mindestens eine oder mehrere Mittelnähte (150) und Seitennähte (160) umfasst,
- wobei die Kontaktschicht (110), die Dispersionsschicht (120) und die mindestens eine Absorptionsschicht (130) mittels der Mittelnähte (150) miteinander verbunden sind, wobei die Mittelnähte (150) nicht durch die undurchlässige Schicht (140) an der Außenseite des wiederverwendbaren Inkontinenzmaterials verlaufen,
- und wobei die Seitennähte (160) die Kontaktschicht (110) und die undurchlässige Schicht (140) miteinander verbinden, ohne durch die Schichten an der Außenseite des Inkontinenzmaterials zu verlaufen, wodurch zwei Außenkanten (170) entstehen, die entlang der Beine eines Trägers angeordnet werden können.

2. Wiederverwendbares
Inkontinenzmaterial (100) nach Anspruch 1, wobei die Seitennähte (160) die nach innen gefalteten Enden der Kontaktschicht (110) und der undurchlässigen Schicht (140) miteinander verbinden.

3. Wiederverwendbares
Inkontinenzmaterial (100) nach einem der vorstehenden Ansprüche, wobei die Absorptionsschicht (130) eine Vliesschicht ist.

4. Wiederverwendbares
Inkontinenzmaterial (100) nach einem der vorstehenden Ansprüche, wobei Seitenränder (121) der Dispersionsschicht (120) zwischen der Dispersionsschicht (120) und der Absorptionsschicht (130) umgeschlagen und mittels der Mittelnaht (150) fest vernäht sind.

5. Wiederverwendbares
Inkontinenzmaterial (100) nach einem der vorstehenden Ansprüche, wobei die Dispersionsschicht (120) und die Absorptionsschicht (130) schmaler sind als der Abstand zwischen den Seitennähten, gemessen über die Kontaktschicht (110) oder die undurchlässige Schicht (140).

6. Wiederverwendbares
Inkontinenzmaterial (100) nach einem der vorstehenden Ansprüche, wobei die undurchlässige Schicht (150) aus einem textilen Material besteht.

7. Wiederverwendbares
Inkontinenzmaterial (100) nach Anspruch 6, wobei das textile Material ein gewebtes oder gewirktes Polyestergewebe umfasst.

8. Wiederverwendbares
Inkontinenzmaterial (100) nach einem der Ansprüche 6 oder 7, wobei das textile Material laminiert ist.

9. Wiederverwendbares
Inkontinenzmaterial (100) nach einem der Ansprüche 6 bis 8, wobei das textile Material mit einer wasserundurchlässigen Membran beschichtet ist.

10. Wiederverwendbares
Inkontinenzmaterial (100) nach einem der vorstehenden Ansprüche, wobei die Absorptionsschicht Polyacrylsäure und Polyestervlies enthält.

11. Wiederverwendbares
Inkontinenzmaterial (100) nach einem der vorstehenden Ansprüche, wobei die Kontaktschicht (110) eine Vliesschicht ist.

12. Wiederverwendbares Inkontinenzmaterial nach einem der vorstehenden Ansprüche, in Form einer Windel, einer Unterhose oder einer Einlage zur Verwendung in einer Unterhose.

13. Verfahren zur Herstellung von wiederverwendbarem Inkontinenzmaterial, das mindestens eine Kontaktschicht (110), die dazu bestimmt ist, mit der Haut in Kontakt zu kommen, eine Dispersionsschicht (120), mindestens eine Absorptionsschicht (130) und eine undurchlässige Schicht (140) umfasst, die dazu bestimmt ist, eine Außenseite des Inkontinenzmaterials zu bilden, wobei das Verfahren umfasst:
- Vernähen der Kontaktschicht (110) mit der Dispersionsschicht (120) und der mindestens einen Absorptionsschicht (130) mittels einer oder mehrerer Mittelnähte (150),
- Vernähen der Kontaktschicht (110) mit der undurchlässigen Schicht (140) entlang zweier Außenkanten dieser Schichten, wobei sich während des Vernähens die Dispersionsschicht und die Absorptionsschicht auf der Außenseite eines Volumens befinden, das von der Kontaktschicht und der undurchlässigen Schicht eingeschlossen ist,
- Drehen der Schichten von innen nach außen, so dass sich die Dispersionsschicht und die Absorptionsschicht auf der Innenseite des Volumens befinden, das von der Kontaktschicht und der undurchlässigen Schicht eingeschlossen ist.

14. Verfahren nach Anspruch 13, wobei die Dispersionsschicht und die Absorptionsschicht schmaler sind als die Kontaktschicht und die undurchlässige Schicht und nicht eingenäht werden, wenn die Kontaktschicht mit der undurchlässigen Schicht vernäht wird.

## Revendications

1. Matériel pour l'incontinence réutilisable (100, 200, 300) ayant un côté interne destiné à être orienté vers le corps d'un porteur, et un côté externe destiné à être orienté à l'opposé du corps d'un porteur, le matériel pour l'incontinence réutilisable comprenant une structure stratifiée, la structure stratifiée comprenant une couche de contact (110), une couche de dispersion (120), au moins une couche d'absorption (130), une couche imperméable (140) au moins une ou plusieurs piqûres intermédiaires (150) et des piqûres latérales (160),
- dans lequel la couche de contact (110), la couche de dispersion (120) et l'au moins une couche d'absorption (130) sont liées les unes aux autres au moyen des piqûres intermédiaires (150), dans lequel les piqûres intermédiaires (150) ne passent pas à travers la couche imperméable (140) au niveau du côté externe du matériel pour l'incontinence réutilisable,
- et dans lequel les piqûres latérales (160) relient entre elles la couche de contact (110) et la couche imperméable (140) sans passer à travers les couches sur le côté externe du matériel pour l'incontinence, donnant lieu à deux bords extérieurs (170) qui peuvent être agencés le long des jambes d'un porteur.

2. Matériel pour l'incontinence réutilisable (100) selon la revendication 1, dans lequel les piqûres latérales (160) relient entre elles des extrémités pliées vers l'intérieur de la couche de contact (110) et de la couche imperméable (140).

3. Matériel pour l'incontinence réutilisable (100) selon l'une quelconque des revendications précédentes, dans lequel la couche d'absorption (130) est une couche de nappe.

4. Matériel pour l'incontinence réutilisable (100) selon l'une des revendications précédentes, dans lequel des bords latéraux (121) de la couche de dispersion (120) sont repliés entre la couche de dispersion (120) et la couche d'absorption (130), et piqués solidement au moyen de la piqûre intermédiaire (150).

5. Matériel pour l'incontinence réutilisable (100) selon l'une quelconque des revendications précédentes, dans lequel la couche de dispersion (120) et la couche d'absorption (130) sont plus étroites que la distance entre les piqûres latérales, mesurées sur la couche de contact (110) ou la couche imperméable (140).

6. Matériel pour l'incontinence réutilisable (100) selon l'une quelconque des revendications précédentes, dans lequel la couche imperméable (150) est constituée d'un matériau textile.

7. Matériel pour l'incontinence réutilisable (100) selon la revendication 6, dans lequel le matériau textile comprend un tissu polyester tissé ou tricoté.

8. Matériel pour l'incontinence réutilisable (100) selon l'une des revendications 6 ou 7, dans lequel le matériau textile est laminé.

9. Matériel pour l'incontinence réutilisable (100) selon l'une des revendications 6 à 8, dans lequel le matériau textile est recouvert d'une membrane imperméable à l'eau.

10. Matériel pour l'incontinence réutilisable (100) selon l'une quelconque des revendications précédentes, dans lequel la couche d'absorption contient de l'acide polyacrylique et du polyester non-tissé.

11. Matériel pour l'incontinence réutilisable (100) selon l'une quelconque des revendications précédentes, dans lequel la couche de contact (110) est une couche de nappe.

12. Matériel pour l'incontinence réutilisable selon l'une quelconque des revendications précédentes, sous forme d'une couche, de sous-vêtements ou d'une serviette pour utilisation dans des sous-vêtements.

13. Procédé de production d'un matériel pour l'incontinence réutilisable qui comprend au moins une couche de contact (110) destinée à venir en contact avec la peau, une couche de dispersion (120), au moins une couche d'absorption (130), et une couche imperméable (140) destinée à former un côté externe du matériel pour l'incontinence, le procédé comprenant :
- la piqûre de la couche de contact (110) sur la couche de dispersion (120) et l'au moins une couche d'absorption (130) au moyen d'une ou plusieurs piqûres intermédiaires (150),
- la piqûre de la couche de contact (110) sur la couche imperméable (140) le long de deux bords extérieurs de ces couches, dans lequel pendant la piqûre la couche de dispersion et la couche d'absorption sont sur l'extérieur d'un volume délimité par la couche de contact et la couche imperméable,
- le retournement des couches de sorte que la couche de dispersion et la couche d'absorption soient sur l'intérieur du volume délimité par la couche de contact et la couche imperméable.

14. Procédé selon la revendication 13, dans lequel la couche de dispersion et la couche d'absorption sont plus étroites que la couche de contact et la couche imperméable, et ne sont pas piquées alors que la couche de contact est piquée sur la couche imperméable.
